## Europäisches Patentamt
## European Patent Office
## Office européen des brevets

(19)

(11) Veröffentlichungsnummer: **0 065 267**
**A2**

## (12) EUROPÄISCHE PATENTANMELDUNG

(21) Anmeldenummer: **82104108.4**

(22) Anmeldetag: **12.05.82**

(51) Int. Cl.³: **C 07 D 493/04**
// (C07D493/04, 307/00, 307/00)

(30) Priorität: **16.05.81 DE 3119553**

(43) Veröffentlichungstag der Anmeldung: **24.11.82**
**Patentblatt 82/47**

(84) Benannte Vertragsstaaten: **BE CH DE FR GB IT LI NL SE**

(71) Anmelder: **HOECHST AKTIENGESELLSCHAFT,
Postfach 80 03 20, D-6230 Frankfurt/Main 80 (DE)**

(72) Erfinder: **Stühler, Herbert, Dr., Hochfelnstrasse 12,
D-8261 Burgkirchen/Alz (DE)**
Erfinder: **Krempl, Engelbert, Hochgernweg 2,
D-8261 Burgkirchen/Alz (DE)**
Erfinder: **Oberhauser, Adelgunde, Untereschlbach 94,
D-8262 Neuötting (DE)**

(54) Verfahren zur Herstellung von Carbonsäureestern von Anhydrohexiten.

(57) Es wird ein Verfahren zur Herstellung von Carbonsäureestern von Anhydrohexiten beschrieben, bei dem Hexit und Carbonsäure im Molverhältnis von 1 : 0,5 bis 1 : 5 in Gegenwart von alkalischen Veresterungskatalysatoren und in Gegenwart einer Inertgasatmosphäre zunächst bei Normaldruck und einer Temperatur von 210 bis 260°C bis zu einer Säurezahl von 2 bis 25 unter Abführen des sich ergebenden Wassers umgesetzt werden und dann die Umsetzung bei Vakuum und einer Temperatur von 210 bis 260°C unter weiterem Abführen des sich ergebenden Wassers fortgeführt wird, worauf das Reaktionsprodukt bei einer Temperatur von 50 bis 100°C mit Wasserstoffperoxid gebleicht wird. Mit diesem Verfahren werden Anhydrohexitcarbonsäureester in guter Farbqualität erhalten. Sie stellen oberflächenaktive Substanzen, insbesondere hochwertige Emulgatoren und Gleitmittel dar.

0065267

HOECHST AKTIENGESELLSCHAFT . HOE 81/F 910     Dr.GL-ba


Verfahren zur Herstellung von Carbonsäureestern von Anhydrohexiten

Die Erfindung betrifft ein Verfahren zur Herstellung von Carbonsäureestern von Anhydrohexiten mit guter Farbqualität.

Anhydrohexite sind intramolekulare Etherverbindungen von Hexiten, die durch Dehydratisierung der Hexite (aliphatische, sechswertige, geradkettige und gesättigte Alkohole mit 6 Kohlenstoffatomen), wie Sorbit, Mannit und Dulcit, gebildet werden. Dabei geht es im wesentlichen um jene Anhydroverbindungen, die aus der intramolekularen Reaktion unter Freisetzung von einem Mol oder von zwei Molen Wasser resultieren. Bei der Entfernung eines Wassermoleküls aus Hexiten entstehen die Monoanhydrohexite, auch Hexitane genannt (zum Beispiel Sorbitan), und bei der Entfernung von zwei Molen Wasser entstehen die Dianhydrohexite, auch Hexide genannt (zum Beispiel Sorbid oder Isosorbit).

Aus der britischen Patentschrift 613 444 ist ein Verfahren zur Herstellung von Carbonsäureestern von Anhydrohexiten bekannt, bei dem zunächst die Anhydroverbindung hergestellt und diese anschließend mit einer Carbonsäure verestert wird. Die Bereitung der intramolekularen Etherverbindung, das ist Mono- oder Dianhydrohexit, erfolgt in der Weise, daß Hexit in Gegenwart von Säuren als Katalysatoren und von einem

0065267

mit Wasser ein Azeotrop bildenden inerten, organischen Lösungsmittel, wie Xylol, auf einer Temperatur von etwa 130 bis 160 °C gehalten wird, wobei das Reaktionswasser durch azeotrope Destillation abgeführt wird. Die so erhaltenen Anhydrohexite werden anschließend mit Carbonsäure umgesetzt, indem Anhydrohexit, Carbonsäure und Veresterungskatalysator, gegebenenfalls in Gegenwart eines inerten, organischen Lösungsmittels, ebenfalls bei etwa 130 bis 160 °C unter Rückfluß und Abführung des Reaktionswassers gekocht wird.

Dieses bekannte Verfahren hat insbesondere den Nachteil, daß es eine sehr lange Reaktionszeit erfordert.

Um diesen Nachteil auszuschalten, wird beim Verfahren der amerikanischen Patentschrift 2 322 820 zur Herstellung von Carbonsäureestern von Anhydrohexiten eine wesentlich höhere Reaktionstemperatur angewandt. Nach diesem Verfahren werden die Anhydrohexitcarbonsäureester hergestellt durch Umsetzung eines Gemisches von Hexit, Carbonsäure und gegebenenfalls Veresterungskatalysator in Gegenwart einer Inertgas-Atmosphäre bei einer Temperatur von 150 bis 300 °C unter Rühren und unter ständigem Abführen des Reaktionswassers. Die Umsetzung wird entweder bei Normaldruck (Atmosphärendruck) oder bei Unterdruck (Vakuum) durchgeführt. Das erhaltene Reaktionsprodukt wird zur Beseitigung von gefärbten Anteilen einer Reinigung mit Aktivkohle unterworfen.

Wird dieses Verfahren bei Normaldruck bei einer Temperatur aus dem unteren Teil des angegebenen Temperaturbereiches durchgeführt, ist eine sehr lange Reaktionszeit erforderlich. Bei höheren Temperaturen ist die Reaktionszeit zwar kürzer, es wird jedoch ein stark dunkel gefärbtes Reaktionsprodukt erhalten, das auch nach der beschriebenen Reinigung mit Aktivkohle noch relativ dunkel gefärbt bleibt. Derart gefärbte Anhydrohexitcarbonsäureester sind weitgehend unbrauchbar. Wird die Umsetzung

- 3 -                    0065267

unter Vakuum vorgenommen, so ist bei den niedrigen
Reaktionstemperaturen ebenfalls eine lange Reaktionszeit erforderlich. Hohe Reaktionstemperaturen haben
in diesem Fall den Nachteil, daß der angestrebte Veresterungsgrad der Anhydrohexite kaum erreicht wird,
weil insbesondere zu Beginn der Umsetzung eine relativ
große Menge an Fettsäure ausgetragen wird. Darüberhinaus ist die technische Durchführung dieser Arbeitsweise nur schwer zu realisieren.

Aufgabe der Erfindung ist es daher, ein Verfahren zur
Herstellung von Carbonsäureestern von Anhydrohexiten
zu schaffen, das insbesondere eine relativ kurze Reaktionszeit erfordert und mit dem Anhydrohexitcarbonsäureester erhalten werden, die nur noch leicht gefärbt
sind, deren Farbe also im allgemeinen nicht wesentlich
stärker hervortritt, als die Farbe der eingesetzten
Carbonsäure.

Es wurde überraschenderweise gefunden, daß diese Aufgabe gelöst werden kann, wenn man Hexit und Carbonsäure
in Gegenwart von alkalischen Veresterungskatalysatoren
zunächst bei Normaldruck auf eine Temperatur von 210
bis 260 °C bis zum Erreichen einer bestimmten Säurezahl
erhitzt und nach Erreichen dieser Säurezahl bei Unterdruck und einer Temperatur von 210 bis 260 °C weiter
erhitzt, wobei das entstehende Reaktionswasser laufend
abgeführt wird, und die so erhaltenen Carbonsäureester
von Anhydrohexiten mit Wasserstoffperoxid bleicht.

Gefunden wurde demnach ein Verfahren zur Herstellung
von Carbonsäureestern von Anhydrohexiten mit guter
Farbqualität durch Umsetzung eines Gemisches von Hexit,
Carbonsäure und alkalischen Veresterungskatalysatoren

.0065267

in Gegenwart einer Inertgas-Atmosphäre bei einer Temperatur von über 200 °C unter Rühren und unter ständiger Entfernung des Reaktionswassers, und Reinigung des Reaktionsproduktes zur Beseitigung von gefärbten Anteilen, dadurch gekennzeichnet, daß man

a) das Gemisch mit einem Molverhältnis von Hexit zu Carbonsäure wie 1 : 0,5 bis 1 : 5 einsetzt,

b) zunächst bei Normaldruck auf einer Temperatur von 210 bis 260 °C hält, bis eine Säurezahl von 2 bis 25 erreicht ist und anschließend unter Vakuum bei einer Temperatur von 210 bis 260 °C hält, bis das aus Anhydrohexit-Bildung und Veresterung sich ergebende Reaktionswasser entfernt ist, und

c) das Reaktionsprodukt bei einer Temperatur von 50 bis 100 °C mit Wasserstoffperoxid behandelt.

Mit dem erfindungsgemäßen Verfahren werden überraschenderweise nur wenig gefärbte Anhydrohexitcarbonsäureester erhalten, obwohl die Reaktionstemperatur ähnlich hoch liegt wie beim Verfahren der amerikanischen Patentschrift 2 322 820, bei dem stark gefärbte Produkte entstehen. Das unerwartete Ergebnis des erfindungsgemäßen Verfahrens resultiert offensichtlich aus der Kombination der angegebenen Verfahrensschritte und insbesondere aus der speziellen Reaktionsführung während der eigentlichen Umsetzung.

Bei den Carbonsäuren, die beim erfindungsgemäßen Verfahren eingesetzt werden, handelt es sich im allgemeinen um aliphatische, geradkettige oder verzweigte, vorzugsweise geradkettige, und gesättigte oder ungesättigte, (vorzugsweise ein- bis dreifach ungesättigte) Monocarbonsäuren.

Bevorzugt werden Fettsäuren, zweckmäßigerweise solche mit 6 bis 30 C-Atomen, vorzugsweise mit 8 bis 22 C-Atomen. Es können auch Gemische von Carbonsäuren, vorzugsweise Fettsäuregemische, eingesetzt werden.

Vorteilhafte Carbonsäuren sind beispielsweise die Hexansäure (Capronsäure), Octansäure (Caprylsäure) Decansäure (Caprinsäure), Dodecansäure (Laurinsäure), Tetradecansäure (Myristinsäure), Hexadecansäure (Palmitinsäure), Octadecansäure (Stearinsäure), Eicosansäure (Arachinsäure), Docosansäure (Behensäure), Dodecensäure (Lauroleinsäure), Tetradecensäure (Myristoleinsäure), Hexadecensäure (Palmitoleinsäure), Octadecensäure (Ölsäure), 12-Oxy-octadecensäure (Ricinolsäure), Octadecadiensäure (Linolsäure) und Octadecatriensäure (Linolensäure) sowie die Cocosnußölsäure, Talgfettsäure und Palmkernfettsäure.

Die Cocosnußölfettsäure weist in der Regel die folgende typische Kettenverteilung in Gewichtsprozent auf: Gesättigt: $C_6$ 0,5 %, $C_8$ 8 %, $C_{10}$ 7 %, $C_{12}$ 48 %, $C_{14}$ 17 %, $C_{16}$ 9 % und $C_{18}$ 2 %; ungesättigt: $C_{16}$ (mit einer Doppelbindung) 0,2 %, $C_{18}$ (mit einer Doppelbindung) 7 % und $C_{18}$ (mit 2 Doppelbindungen) 1,3 %.

Die Talgfettsäure weist in der Regel die folgende typische Kettenverteilung auf: Gesättigt: $C_{12}$ 0,5 %, $C_{14}$ 3 %, $C_{15}$ 1 %, $C_{16}$ 25 %, $C_{17}$ 2 %, $C_{18}$ 19 % und $C_{20}$ 1 %; ungesättigt: $C_{14}$ (mit einer Doppelbindung) 0,5 %, $C_{16}$ (mit einer Doppelbindung) 3 %, $C_{18}$ (mit einer Doppelbindung) 40 %, $C_{18}$ (mit zwei Doppelbindungen) 4 % und $C_{18}$ (mit drei Doppelbindungen) 1 %.

Die Palmkernfettsäure weist in der Regel die folgende typische Kettenverteilung in Gewichtsprozent auf:

Gesättigt: $C_6$ 0,5 %, $C_8$ 4 %, $C_{10}$ 5 %, $C_{12}$ 50 %, $C_{14}$ 15 %, $C_{16}$ 7 % und $C_{18}$ 2 %; ungesättigt: $C_{16}$ (mit einer Doppelbindung) 0,5 %, $C_{18}$ (mit einer Doppelbindung) 15 % und $C_{18}$ (mit zwei Doppelbindungen) 1 %. Die Carbonsäuren werden in der Regel als solche, das heißt ohne jegliche Lösungsmittel, eingesetzt.

Von den Hexahydroalkoholen, den Hexiten, werden vorzugsweise Sorbit, Mannit, und/oder Dulcit eingesetzt. Ähnlich wie bei den Carbonsäuren wird auch hier in der Regel jene Form verwendet, die im allgemeinen vorliegt. Mannit und Dulcit sind beispielsweise in fester Form erhältlich und werden daher als solche eingesetzt. Sorbit ist entweder fest oder als wäßrige Lösung (Sirup) erhältlich. Er kann in beiden Formen verwendet werden. Im Fall der Verwendung von Sorbit als wäßrige Lösung wird das Lösungswasser bereits beim Erhitzen auf die angegebene Reaktionstemperatur ausgetragen.

Das Molverhältnis von Hexit zu Carbonsäure beträgt erfindungsgemäß 1 : 0,5 bis 1 : 5, vorzugsweise 1 : 1 bis 1 : 4.

Bei der erfindungsgemäßen Umsetzung werden alkalische Katalysatoren eingesetzt.
Die Menge an Katalysator kann in weiten Grenzen variieren. Sie beträgt im allgemeinen 0,01 bis 1,5 Gew.-%, vorzugsweise 0,1 bis 1 Gew.-%, bezogen auf das Gewicht der Carbonsäure.
Als alkalische (basische) Katalysatoren können beispielsweise Alkali- oder Erdalkali-Hydroxide, -Carbonate, -Alkoholate mit einer Alkylgruppe mit 1 bis 4 C-Atomen und Alkali- oder Erdalkali-Oxide einzeln oder Mischungen davon, eingesetzt werden. Bevorzugt sind, einzeln oder in Mischung, Alkalihydroxide wie Ätzkali und Ätznatron, Alkalicarbonate wie Kalium- und Natriumcarbonat, und Alkalialkoholate mit einer Alkylgruppe mit 1 bis 2 C-Atomen wie Kalium- und Natriummethylat.

Als Inertgase kommen solche Gase in Betracht, die mit den Ausgangssubstanzen und dem Reaktionsprodukt keine Reaktion eingehen. Bevorzugt sind Stickstoff, Kohlendioxid und Edelgase.

Die Inertgas-Atmosphäre wird beim erfindungsgemäßen Verfahren vorzugsweise dadurch erzeugt, daß ein Inertgasstrom über oder durch die Reaktionsmischung geleitet wird. Er beträgt im allgemeinen pro Kilogramm Reaktionsmischung 0,5 bis 20 Liter pro Stunde, vorzugsweise 1 bis 15 Liter pro Stunde.

Beim erfindungsgemäßen Verfahren wird das Gemisch aus Hexit, Carbonsäure und Katalysator möglichst rasch, vorzugsweise in einer Zeit von höchstens 20 bis 60 Minuten auf die Reaktionstemperatur gebracht.

Die Reaktionstemperatur beträgt 210 bis 260 °C, vorzugsweise 220 bis 240 °C.

Erfindungsgemäß wird zunächst bei Normaldruck (Atmosphärendruck) bis zu einer Säurezahl von 2 bis 25, vorzugsweise von 3 bis 15, bei Reaktionstemperatur erhitzt und das Reaktionswasser abgeführt. Das Wasser wird bereits aufgrund der hohen Reaktionstemperatur aus dem Reaktionssystem entfernt. Der genannte Inertgasstrom bewirkt ebenfalls die Austragung des vorhandenen Wassers.

Bei einer Säurezahl von weniger als 2 wären relativ lange Reaktionszeiten erforderlich und das Reaktionsprodukt wäre selbst nach der Behandlung mit Wasserstoffperoxid farblich noch unbefriedigend. Mit Säurezahlen von mehr als 25 würden nur unwirtschaftliche Ausbeuten erreicht.

Es hat sich als vorteilhaft erwiesen, wenn die Säurezahl möglichst rasch, vorzugsweise in einer Zeit von höchstens 30 bis 120 Minuten erreicht ist.

Nachdem die angegebene Säurezahl erreicht ist (das Reaktionsgemisch, das zunächst mehrphasig war, ist nun homogen), wird an das Reaktionssystem Vakuum angelegt. Das Vakuum kann in weiten Grenzen variieren. Seine Grenze liegt lediglich dort, wo auch Reaktionskomponenten oder Reaktionsprodukte ausgetragen werden könnten. Zweckmäßigerweise wird ein solches Vakuum eingestellt, daß das Wasser zügig ausgetragen wird. Es liegt demnach im allgemeinen bei 1000 bis 5000 Pa vorzugsweise bei 2000 bis 4000 Pa. Die Reaktionstemperatur bleibt bei 210 bis 260 °C, vorzugsweise bei 220 bis 240 °C. Die Reaktion wird unter Vakuum solange fortgeführt, bis die gewünschte Menge an Reaktionswasser, das ist die rechnerisch feststellbare Wassermenge, die sich aus der Anhydrohexit-Bildung und Veresterung ergibt, entfernt ist (die Beendigung der Umsetzung ist daran zu erkennen, daß vergleichsweise nur mehr wenig Wasser pro Zeiteinheit entwickelt und ausgetragen wird).

Nach Beendigung der Umsetzung wird das Reaktionsprodukt möglichst rasch, vorzugsweise in einer Zeit von höchstens 20 bis 60 Minuten, abgekühlt, zweckmäßigerweise auf eine Temperatur von 50 bis 100 °C, vorzugsweise 60 bis 80 °C.

Die Reaktionszeit des erfindungsgemäßen Verfahrens hängt insbesondere von der gewählten Temperatur und von dem gewählten Molverhältnis von Hexit und Carbonsäure ab. Die gesamte Reaktionszeit beträgt im allgemeinen 4 bis 6 Stunden. Liegt im Gemisch von Hexit und Carbonsäure relativ viel Carbonsäure vor, ist der untere Bereich der angegebenen Zeit erreichbar. Bei relativ wenig Carbonsäure sind längere Reaktionszeiten erforderlich. Die Reaktionszeit ist also insbesondere im Fall von Molverhältnissen von Hexit und Carbonsäure wie 1 : 3 bis 1 : 5 relativ kurz.

Nach Beendigung der Umsetzung wird das Reaktionsprodukt
bei einer Temperatur von 50 bis 100 °C, vorzugsweise
von 60 bis 80 °C, mit Wasserstoffperoxid behandelt.
Die Behandlung (Bleichung) mit Wasserstoffperoxid erfolgt durch Inkontaktbringen des Reaktionsproduktes
mit Wasserstoffperoxid. Dadurch wird eine derart weitgehende Aufhellung (Bleichung) des zunächst braun bis
dunkelbraun gefärbten Reaktionsproduktes erreicht, daß
es nur noch wenig gefärbt ist.

Die Bleichung wird zweckmäßigerweise so durchgeführt,
daß dem Reaktionsprodukt bei einer Temperatur von 50
bis 100 °C, vorzugsweise von 60 bis 80 °C, eine solche
Menge Wasserstoffperoxid zugesetzt wird, daß nach dem
Zusetzen und nach Rühren bei der genannten Temperatur
bis zu etwa einer Stunde keine weitere Aufhellung mehr
erreicht werden kann. Das Wasserstoffperoxid wird
zweckmäßigerweise in einer wäßrigen Lösung eingesetzt.
Die Behandlungszeit beträgt im allgemeinen 5 bis 60 Minuten, vorzugsweise 10 bis 30 Minuten. Sie ist relativ
kurz bei der Verwendung von konzentrierten Lösungen,
und relativ lang bei der Verwendung von verdünnten
Wasserstoffperoxid-Lösungen. Da das im Handel erhältliche Wasserstoffperoxid eine etwa 35 gew.-%ige wäßrige
Lösung ist, wird zur erfindungsgemäßen Bleichung vorzugsweise diese Lösung eingesetzt.

Nach der Bleichung wird im allgemeinen das eingebrachte
Wasser zweckmäßigerweise durch Destillation entfernt
und der Rückstand (das Reaktionsprodukt) noch filtriert
oder abgenutscht.

Nach einer bevorzugten Ausführung wird die Bleichung
in der Weise durchgeführt, daß das Reaktionsprodukt
bei einer Temperatur von 50 bis 100 °C, vorzugsweise
60 bis 80 °C, mit etwa 0,5 bis 5 Gew.-%, vorzugsweise
etwa 1 bis 3 Gew.-%, bezogen auf das Gewicht des Reaktionsproduktes, einer etwa 35 gew.-%igen wäßrigen Wasserstoffperoxid-Lösung versetzt und etwa 10 bis 30 Minuten lang bei der genannten Temperatur gerührt wird, worauf das durch die Wasserstoffperoxid-Behandlung eingebrachte Wasser abdestilliert und der Rückstand (das Reaktionsprodukt) zweckmäßigerweise noch filtriert oder abgenutscht wird. Das Abdestillieren des Wassers erfolgt vorzugsweise bei einer Temperatur von 60 bis 120 °C und einem Vakuum von 500 bis 2000 Pa.

Das Reaktionsprodukt des erfindungsgemäßen Verfahrens stellt eine mehr oder weniger viskose Flüssigkeit oder einen Feststoff dar und besteht in der Regel aus einer Mischung von einzelnen Anhydrohexitcarbonsäureestern. Es enthält je nach Reaktionsbedingungen und Molverhältnis von Hexit zu Carbonsäure hauptsächlich Hexitanmono-, Hexitandi-, Hexitantri- und Hexitantetracarbonsäureester sowie die entsprechenden Hexidcarbonsäureester. Da das Reaktionsprodukt bereits als solches vielseitig verwendbar ist, erübrigt sich im allgemeinen eine Isolierung von einzelnen Estern oder von Gruppen von Estern. Es kann aber zweckmäßig sein, im Produkt befindliche Katalysatoren und gegebenenfalls nicht veresterte Anhydrohexite, beispielsweise durch Waschen mit wäßrigen Lösungen von Salzen wie Natriumchlorid und Natriumsulfat, zu entfernen. Mit dem erfindungsgemäßen Verfahren werden Carbonsäureester von Anhydrohexiten erhalten, die nur relativ wenig gefärbt sind. Sie weisen im allgemeinen keine stärkere Färbung auf, als die Eigenfarbe der eingesetzten Carbonsäure.

Die mit dem erfindungsgemäßen Verfahren hergestellten Carbonsäureester von Anhydrohexiten sind wertvolle oberflächenaktive Substanzen, insbesondere Emulgatoren und Gleitmittel. Sie werden vorteilhaft beispielsweise zur Herstellung von Wasser-in-Öl- oder Öl-in-Wasser-Emulsionen, bei der Formulierung von kosmetischen Präparaten, in der Waschmittelindustrie sowie als Gleitmittel in der Kunststoffverarbeitung eingesetzt. Bei diesen oder anderen Verwendungsarten kann es unter Umständen zweckmäßig sein, die oberflächenaktiven Eigenschaften der erfindungsgemäß hergestellten Carbonsäureester zu variieren, beispielsweise durch Ethoxylierung und/oder Propoxylierung.

Die Erfindung wird nun an Beispielen noch näher erläutert:

Beispiel. 1

In einen mit Thermometer, Rührer und Wasserabscheide-Vorrichtung und einer Vorrichtung zum Durchleiten eines Inertgases sowie mit einem Vakuumanschluß ausgestatteten 2-Liter-Reaktionsgefäß wurden 3 mol (846 g) Ölsäure, 2 mol (364 g) pulverförmiges Sorbit und 4,8 g Ätznatron eingebracht und durch Rühren vermischt.

Unter Weiterrühren und Durchleiten eines Stickstoffstromes von etwa 2 Liter pro Stunde wurde in 25 Minuten auf 220 °C erhitzt, wobei Sorbit in den geschmolzenen Zustand überging und die Abscheidung von Reaktionswasser einsetzte. Das Wasser wurde über die Wasserabscheide-Einrichtung, durch die auch der Inertgas-Strom floß, abgeführt.

Nachdem 1 1/2 Stunden auf 220 °C erhitzt worden war, betrug die Säurezahl 3,1. Nun wurde das Reaktionsgefäß mit Hilfe des Vakuumanschlusses unter ein Vakuum von etwa 3000 Pa gesetzt. Die Temperatur war weiterhin 220 °C. Rührung und Stickstoff-Strom liefen weiter. Das Reaktionswasser ging laufend ab.

Nach 4 Stunden Reaktionszeit (Gesamtreaktionszeit) waren 100 ml Wasser ausgetragen. In den letzten 20 Minuten ging nur noch sehr wenig Wasser ab, was auf die Beendigung der Umsetzung hinwies. Das Reaktionsprodukt wurde nun innerhalb von 30 Minuten auf etwa 80 °C abgekühlt.

Dem Reaktionsprodukt (1110 g) wurden nun 22 g (2 Gew.-%) von einer wäßrigen, 35 gew.-%igen Wasserstoffperoxid-Lösung zugesetzt und anschließend 30 Minuten lang bei 80 °C gerührt. Dann wurde das durch die Bleichung vorhandene Wasser (Lösungswasser und Reaktionswasser) durch Destillation bei 100 °C und einem Vakuum von 2000 Pa entfernt.

Das so erhaltene Reaktionsprodukt, eine Flüssigkeit mit mittlerer Viskosität, besaß die folgenden Eigenschaften:

0065267

Iodfarbzahl : 12
Säurezahl : 4,5
Hydroxylzahl: 197
Viskosität bei 25 °C: 1600 mPa · s.

### Beispiel 2

In der in Beispiel 1 beschriebenen Apparatur wurde zunächst 1 mol (201 g) Laurinsäure vorgelegt und durch Erhitzen auf etwa 100 °C geschmolzen. Nun wurden 1 mol (182 g) Sorbit, in Form einer 80 %igen wäßrigen Lösung, und 1,5 g Ätznatron eingebracht.
Unter Rühren und Durchleiten von Stickstoff (5 l/h) wurde innerhalb von 30 Minuten auf 240 °C erhitzt und diese Temperatur bei Atmosphärendruck aufrechterhalten.

Als nach 1 1/2 Stunden eine Säurezahl von 7,3 erreicht war, wurde ein Vakuum von 3000 Pa angelegt. Die Temperatur betrug weiterhin 240 °C. Rührung und Stickstoffstrom liefen weiter. Reaktionswasser ging laufend ab. Nach 4 1/2 Stunden Reaktionszeit (Gesamtreaktionszeit) waren 40 ml Wasser ausgetragen. Da das Ende der Umsetzung erkennbar war, wurde der Inhalt des Reaktionsgefäßes innerhalb von 20 Minuten auf 80 °C gekühlt. Dem Reaktionsprodukt (343 g) wurden nun 6,8 g (2 Gew.-%) einer 35 gew.-%igen, wäßrigen Wasserstoffperoxid-Lösung zugesetzt und anschließend 30 Minuten bei 80 °C gerührt. Dann wurde das durch die Bleichung vorhandene Wasser (Lösungswasser und Reaktionswasser) durch Destillation bei 100 °C und einem Vakuum von 2000 Pa entfernt. Das so erhaltene Reaktionsprodukt, eine hochviskose Flüssigkeit, besaß die folgenden Eigenschaften:

Iodfarbzahl : 10
Säurezahl : 6,7
Hydroxylzahl : 340
Viskosität bei 25 °C: 4250 mPa · s.

Beispiel 3

In der in Beispiel 1 beschriebenen Apparatur wurden
1 mol (271 g) Stearinsäure, 1 mol (182 g) Sorbit, in
gekörnter Form, und 2,5 g Ätzkali vorgelegt und durch
Erhitzen auf etwa 100 °C geschmolzen.
Unter Durchleiten von Stickstoff (5 l/h) wurde innerhalb von 25 Minuten auf 220 °C erhitzt und diese Temperatur 2 Stunden lang bei Atmosphärendruck aufrechterhalten. Das Reaktionswasser wurde über die Wasserabscheide-Vorrichtung ständig abgeführt.
Als nach 2 Stunden die Säurezahl 15 erreicht war, wurde
ein Vakuum von 4000 Pa angelegt und unter Rühren und
Stickstoffstrom 4 Stunden weitergefahren. Dann waren
25 ml Wasser ausgetragen. Das Reaktionsprodukt wurde
nun innerhalb von 20 Minuten auf etwa 80 °C abgekühlt.
Dem Reaktionsprodukt (428 g) wurden nun 4,3 g (1 Gew.-%)
von einer 35 gew.-%igen, wäßrigen Wasserstoffperoxid-
Lösung zugesetzt und anschließend 30 Minuten bei 80 °C
gerührt. Dann wurde das durch die Bleichung vorhandene
Wasser (Lösungswasser und Reaktionswasser) durch Destillation bei 100 °C und einem Vakuum von 2000 Pa entfernt.
Das so erhaltene Reaktionsprodukt, ein wachsartiger Feststoff, besaß die folgenden Eigenschaften:

Iodfarbzahl   :   14
Säurezahl     :    8
Hydroxylzahl  :  284.


Beispiel 4

In der in Beispiel 1 beschriebenen Apparatur wurden
3 mol (846 g) Ölsäure, 1 mol (182 g) Sorbit, in Form
einer 80 %igen wäßrigen Lösung, und 4,1 g Ätznatron
vorgelegt.
Unter Durchleiten eines Stickstoffstromes von 15 l/h und
unter Rühren wurde in 25 Minuten auf 220 °C erhitzt, wobei die Abscheidung von Wasser einsetzte. Die Temperatur von 220 °C wurde 1 1/2 Stunden bei Atmosphärendruck beibehalten, die Säurezahl betrug dann 12.

Es wurde ein Vakuum von 3000 Pa angelegt, und Rühren und Stickstoffstrom beibehalten. Nach 4 Stunden Gesamtreaktionszeit waren 64 ml Wasser ausgetragen.

Das Reaktionsprodukt wurde nun in 25 Minuten auf ca. 80 °C abgekühlt.

Dem Reaktionsprodukt (904 g) wurden 4,5 g (0,5 Gew.-%) von einer 35 gew.-%igen, wäßrigen Wasserstoffperoxid-Lösung zugesetzt und anschließend 30 Minuten bei 80 °C gerührt. Dann wurde das durch die Bleichung vorhandene Wasser (Reaktionswasser und Lösungswasser) durch Destillation bei 100 °C und einem Vakuum von 2000 Pa entfernt.

Das so erhaltene Reaktionsprodukt ist eine mittelviskose Flüssigkeit mit folgenden Eigenschaften:

Iodfarbzahl   :    16
Säurezahl     :     8
Hydroxylzahl  :    70
Viskosität bei 25 °C: 200 mPa · s.


Beispiel 5

In der in Beispiel 1 beschriebenen Apparatur wurden 3 mol (924 g) Isostearinsäure vorgelegt, auf etwa 100 °C erhitzt und dann 2 mol (384 g) pulverförmiges Sorbit und 5,2 g Ätznatron eingebracht und geschmolzen. Unter Rühren und Durchleiten eines Stickstoffstromes von 12 l/h wurde innerhalb von 30 Minuten auf 220 °C erhitzt und 1 1/2 Stunden lang bei Atmosphärendruck gehalten. Reaktionswasser wurde laufend abgeführt. Nach 1 1/2 Stunden betrug die Säurezahl 3.

Nun wurde ein Vakuum von 4000 Pa angelegt. Nach 4 Stunden Gesamtreaktionszeit waren 100 ml Wasser ausgetragen, das Produkt wurde nun in 20 Minuten auf 80 °C gekühlt.

0065267

Dem Reaktionsprodukt (1208 g) wurden 24 g (2 Gew.-%) von einer 35 gew.-%igen, wäßrigen Wasserstoffperoxid-Lösung zugesetzt und anschließend 30 Minuten bei 80 °C gerührt. Dann wurde das durch die Bleichung eingebrachte Wasser durch Destillation bei 100 °C und einem Vakuum von 2000 Pa entfernt.

Das so erhaltene Reaktionsprodukt, eine Flüssigkeit mit mittlerer Viskosität, besaß die folgenden Eigenschaften:

Iodfarbzahl   :   8
Säurezahl     :   10
Hydroxylzahl  :  181
Viskosität bei 25 °C: 1500 mPa·s.

Die Bestimmung der in den Beispielen zur Charakterisierung der hergestellten Anhydrohexitcarbonsäureester angegebenen Kennzahlen erfolgte nach den "Einheitsmethoden der deutschen Gesellschaft für Fettwissenschaft".

Patentansprüche

1. Verfahren zur Herstellung von Carbonsäureestern von Anhydrohexiten mit guter Farbqualität durch Umsetzung eines Gemisches von Hexit, Carbonsäure und alkalischen Veresterungskatalysatoren in Gegenwart einer Inertgas-Atmosphäre bei einer Temperatur von über 200 °C unter Rühren und unter ständiger Entfernung des Reaktionswassers, und Reinigung des Reaktionsproduktes zur Beseitigung von gefärbten Anteilen, dadurch gekennzeichnet, daß man

a) das Gemisch mit einem Molverhältnis von Hexit zu Carbonsäure wie 1 : 0,5 bis 1 : 5 einsetzt,

b) zunächst bei Normaldruck auf einer Temperatur von 210 bis 260 °C hält, bis eine Säurezahl von 2 bis 25 erreicht ist und anschließend unter Vakuum bei einer Temperatur von 210 bis 260 °C hält, bis das aus Anhydrohexit-Bildung und Veresterung sich ergebende Reaktionswasser entfernt ist, und

c) das Reaktionsprodukt bei einer Temperatur von 50 bis 100 °C mit Wasserstoffperoxid behandelt.

2. Verfahren nach Anspruch 1, dadurch gekennzeichnet, daß Hexit und Carbonsäure in einem Molverhältnis von 1 : 1 bis 1 : 4 eingesetzt werden.

3. Verfahren nach Anspruch 1, dadurch gekennzeichnet, daß als Hexite Sorbit oder Mannit und als Carbonsäuren Fettsäuren eingesetzt werden.

4. Verfahren nach Anspruch 1, dadurch gekennzeichnet, daß als Katalysator Alkalihydroxide, Alkalicarbonate und/oder Alkalialkoholate mit einer Alkylgruppe mit 1 bis 2 C-Atomen eingesetzt werden.

0065267

5. Verfahren nach Anspruch 1, dadurch gekennzeichnet,
daß bei Normaldruck bis zu einer Säurezahl von
3 bis 15 umgesetzt wird.

6. Verfahren nach Anspruch 1, dadurch gekennzeichnet,
daß bei einem Vakuum von 1000 bis 5000 Pa umgesetzt
wird.

7. Verfahren nach Anspruch 1, dadurch gekennzeichnet,
daß eine Reaktionstemperatur von 220 bis 240 °C eingehalten wird.

8. Verfahren nach Anspruch 1, dadurch gekennzeichnet,
daß das Reaktionsprodukt bei einer Temperatur von
50 bis 100 °C mit 0,5 bis 5 Gew.-%, bezogen auf das
Gewicht des Reaktionsproduktes, von einer etwa
35 gew.-%igen wäßrigen Wasserstoffperoxid-Lösung versetzt und 10 bis 30 Minuten lang bei der genannten
Temperatur gerührt wird, worauf das durch die Wasserstoffperoxid-Behandlung eingebrachte Wasser abdestilliert wird.

9. Verfahren nach Anspruch 1, dadurch gekennzeichnet,
daß man

a) Sorbit oder Mannit und Fettsäure im Molverhältnis
von 1 : 1 bis 1 : 4 und einen alkalischen Katalysator ausgewählt aus der Gruppe bestehend aus
Kaliumhydroxid, Natriumhydroxid, Kaliumcarbonat,
Natriumcarbonat, Kaliumethylat und Natriummethylat
einsetzt,

b) unter Rühren bei Normaldruck in einer Zeit von
höchstens 20 bis 60 Minuten auf eine Temperatur
von 220 bis 240 °C erhitzt und bei dieser Temperatur bis zum Erreichen einer Säurezahl von 3 bis 15
hält,

0065267

c) nach Erreichen der Säurezahl die Umsetzung mit einem Vakuum von 2000 bis 4000 Pa zu Ende führt,

d) das Reaktionsprodukt in einer Zeit von höchstens 20 bis 60 Minuten auf 60 bis 80 °C abkühlt, und

e) das Reaktionsprodukt bei einer Temperatur von 60 bis 80 °C mit 1 bis 3 Gew.-%, bezogen auf das Gewicht des Reaktionsproduktes, von einer etwa 35 gew.-%igen wäßrigen Wasserstoffperoxid-Lösung versetzt und 10 bis 30 Minuten lang bei der genannten Temperatur rührt, worauf man das durch die Wasserstoffperoxid-Behandlung eingebrachte Wasser bei einer Temperatur von 60 bis 120 °C und einem Vakuum von 500 bis 2000 Pa abdestilliert.